(19)

Europäisches
Patentamt
European
Patent Office
Office européen
des brevets

(11) **EP 3 527 122 A1**

(12) **EUROPEAN PATENT APPLICATION**

(43) Date of publication:
**21.08.2019 Bulletin 2019/34**

(51) Int Cl.:
*A61B 5/00* (2006.01)    *A61B 5/024* (2006.01)

(21) Application number: **18156677.9**

(22) Date of filing: **14.02.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(71) Applicant: **Koninklijke Philips N.V.**
**5656 AE Eindhoven (NL)**

(72) Inventors:
• **De Andrade Serra, P.J.**
  **5656 AE Eindhoven (NL)**
• **Regis, Marta**
  **5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **MODELLING AND EXTRACTING INFORMATION FROM A PHOTOPLETHYSMOGRAPHY, PPG, SIGNAL**

(57)    A method of modelling and extracting information from a photoplethysmography, PPG, signal comprises decomposing and modelling (102) the PPG signal as a long-term periodic component and a short-term periodic component. The method further comprises summarizing (104) the information contained in the PPG signal, based on a distribution of fitted parameters of the modelled long-term and short-term periodic components.

Fig. 1

EP 3 527 122 A1

## Description

FIELD OF THE INVENTION

The invention relates to a system and method for modelling and extracting information from a photoplethysmography, PPG, signal.

BACKGROUND OF THE INVENTION

The general background of the invention is in photoplethysmography, PPG, signals. Being located far from the heart, photoplethysmography (PPG) signals carry information not only about the heart rate and power, but also about pressure at the device location, movement, arterial elasticity, blood pressure, peripheral skin perfusion and respiration among the others. PPG signals can be used for extensive analyses of the health status in a broader sense. On the one hand this is what makes the PPG an extremely rich signal, on the other hand this is also the cause of its complexity.

In fact, modelling the PPG signal and understanding its components is still a challenging open problem. Most of the development so far has focused on retrieving isolated information, e.g. heart rate and respiration rate.

Furthermore, the methods proposed may be difficult to interpret (e.g. high order autoregression (Tarassenko & Fleming, 2009)) or may provide only partial understanding of the physiological information contained in PPG (e.g. respiration (Townsend & Collins, 2003).

SUMMARY OF THE INVENTION

As described above, current methods of analyzing PPG signals may be limited in the amount of information they extract and the complexity of the resulting data may make them difficult to interpret. Furthermore, in more comprehensive methods to extract and monitor various physiological values from PPG signals (see for example, Dekker, 2003), inference is generally not based on shape (e.g. such methods may not consider the shape profiles of pulses in the PPG signal). In fact, models (e.g. existing models) addressing the shape have the limitation of assuming it deterministic (e.g. assuming PPG pulses are of a particular shape and can be modelled using a particular parametric function).

Furthermore, most of the approaches proposed so far either fail in case of noise or pathological conditions, or are specifically suited to some disorders (e.g. arrhythmia), and thus applicable only to selected categories of patients.

The methods and systems described herein aim to improve upon some of these issues and others. The present application describes an innovative method to model and extract information from a photoplethysmography (PPG) signal. In some example embodiments a two-step approach will be described, decomposing and modelling the long-term and short-term periodic components separately.

In some embodiments, the resulting system is self-contained, automated and provides a good compression of the data while keeping the rich information of the PPG signal. The user can tune the parameters in order to achieve greater fitting accuracy or higher compression of the data. The output of the algorithm provides the distribution of the fitted parameters, rather than simple estimates, and thus summarizes the information contained in the PPG signal. The method is implementable, for example, in devices for real time unobtrusive and continuous monitoring, possibly in combination with other measurements. It finds thus application, among the others, in the diagnosis of various disorders.

According to a first aspect there is provided a computer implemented method of modelling and extracting information from a photoplethysmography, PPG, signal. The method comprises decomposing and modelling the PPG signal as a long-term periodic component and a short-term periodic component and summarizing the information contained in the PPG signal, based on a distribution of fitted parameters of the modelled long-term and short-term periodic components.

In this way, a data rich PPG signal can be compressed and reduced to parameters associated with a model of the long-term component and parameters associated with a short-term component of the signal. This reduces the computation storage space needed to store the PPG signal, yet, as will be shown below, still allows for a detailed and highly accurate reconstruction of the original PPG signal to be made. Furthermore, the parameters output from the modelling enables monitoring and diagnosis of abnormalities.

According to a second aspect there is provided a system configured for modelling and extracting information from a photoplethysmography, PPG, signal. The system is configured to decompose and model the PPG signal as a long-term periodic component and a short-term periodic component and summarize the information contained in the PPG signal, based on a distribution of fitted parameters of the modelled long-term and short-term periodic components.

According to a third aspect there is provided a computer program product comprising a non-transitory computer readable medium, the computer readable medium having computer readable code embodied therein, the computer readable code being configured such that, on execution by a suitable computer or processor, the computer or processor is caused to perform the method above.

These and other aspects of the invention will be apparent from and elucidated with reference to the embodiments described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 shows an example method according an embodiment;
Fig. 2a-2f show example plots illustrating how a PPG signal may be decomposed and modelled as a long-term periodic component and a short-term periodic component according to some embodiments;
Fig. 3 shows an example method according to some embodiments;
Fig. 4 shows an example system according to some embodiments;
Fig. 5 shows an example method according to some embodiments;
Fig. 6 shows example fits to a PPG signal according to some embodiments;
Fig. 7 shows example fits of a PPG signal from a patient with atrial fibrillation according to some embodiments;
Fig. 8 shows example box plots of shape parameters according to some embodiments;
Fig. 9 shows 95% prediction intervals of pulse shapes according to some embodiments;
Fig. 10 shows variances and cross-correlations between shape parameters for non-atrial fibrillation according to embodiments herein;
Fig. 11 shows variances and cross-correlations between shape parameters for atrial fibrillation according to embodiments herein;
Figs 12-25 show enlarged versions of the plots shown in Figs 2a-2f, 6, 7, 8 and 9 respectively.

DETAILED DESCRIPTION OF EMBODIMENTS

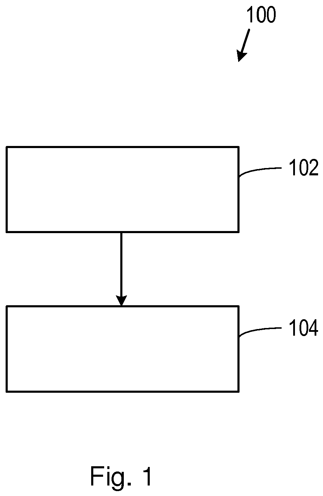

Fig. 1 shows a computer implemented method 100 of modelling and extracting information from a photoplethysmography (PPG) signal according to some embodiments herein. Briefly, in a first step 102 the method comprises decomposing and modelling the PPG signal as a long-term periodic component and a short-term periodic component. In a second step 104 the method comprises summarizing the information contained in the PPG signal, based on a distribution of fitted parameters of the modelled long-term and short-term periodic components.
The skilled person will be familiar with photoplethysmography and PPG signals, but in brief, a PPG signal describes volumetric changes (e.g. changes in blood volume) of an organ. A PPG signal may be measured, for example, using a pulse oximeter which illuminates the skin and measures changes in light absorption. The skilled person may be familiar with other methods of measuring a PPG signal and the methods described herein will be understood to also apply thereto.

Generally, as described herein, the term "long-term periodic component" refers to an envelope of the PPG signal (for example, cyclic changes in the pulses of the PPG signal due to, for example, respiration). The long-term periodic component may also be referred to herein as the "first component" or Component I.
Generally, as described herein, the term "short-term periodic component" refers to the individual pulses of the PPG signal (for example, cyclic changes in blood volume, due to cardiac cycles). The short-term periodic component may also be referred to herein as the "second component" or Component II.
Below we list examples of innovative aspects of the proposed designs, as well as how they overcome some problems and disadvantages of the state of the art:

1) Component-based - the automatic separation of the signal into components allows, in some embodiments, the investigation of multiple physiological processes at the same time and their interaction, for a comprehensive understanding of the underlying physiology and of the health status
2) Curve registration - in some embodiments with this functional approach we align the curves corresponding to different pulses in the PPG before estimating their shape. This avoids a need to truncate the curves, and preserves all the physiological information contained in the variable length of the PPG pulses
3) Fast iterative process - Can be implemented, for example, in devices for real time analyses, using relevant information available
4) Random shape of the pulses - PPG pulses are typically modelled as having a deterministic and/ or parametric shape. Deviations from this deterministic and/or parametric shape are then modelled as noise. Some of the embodiments proposed assume instead that the pulses are random and we derive distributional information. From this information it is possible to produce statements about the uncertainty associated with point estimates

- Distribution - the distribution of the shape parameters is a natural output of the model. From this, statistical information can be extracted and compared between people and/or groups of people.
- Correlations - the correlations between shape parameters can be analyzed in order to get an insight into the pulses.

5) Respiration - Previous literature uses the traditional three components to estimate respiration rate (Pimentel, Charlton, & Clifton, 2015). Some of the embodiments described herein approach the respiration differently, modelling fully the long-term periodic component.
6) Robust to various conditions of the patients - can thus be used for monitoring and diagnosis.

In more detail with respect to Fig. 1, in some embodiments, the step of decomposing and modelling 102 of Fig. 1 may comprise splitting the PPG signal into the long-term periodic component and the short-term periodic component. In some embodiments the step of decomposing and modelling 102 may comprise modelling the long-term periodic component and the short-term periodic component. In some embodiments decomposing and modelling 102 may be performed as one step (e.g. after modelling the long-term periodic component, the long-term periodic component may be removed from the signal leaving the short-term periodic component.)

As noted above, in some embodiments the long-term periodic component comprises an envelope of the PPG signal.

In some embodiments, the step of decomposing and modelling 102 comprises modelling the long-term periodic component using a non-parametric function such as a spline function (e.g. a linear piecewise spline) or a wavelet function. The use of non-parametric functions means that no predetermined shape is assumed for the long-term periodic component. This may be more flexible and accurate compared to fitting parametric functions to an envelope of the PPG because parametric functions assume a specific shape for the envelope (e.g. such as a Gaussian shape) and are thus less flexible and less accurate.

In some embodiments, the step of decomposing and modelling 102 comprises fitting a spline function to one or more maxima or one or more minima of individual pules in the PPG signal. As such, the method 100 may further comprise determining the location of one or more minima and/or maxima in the PPG signal and fitting a spline function to the one or more determined maxima or the one or more determined minima of the individual pules in the PPG signal.

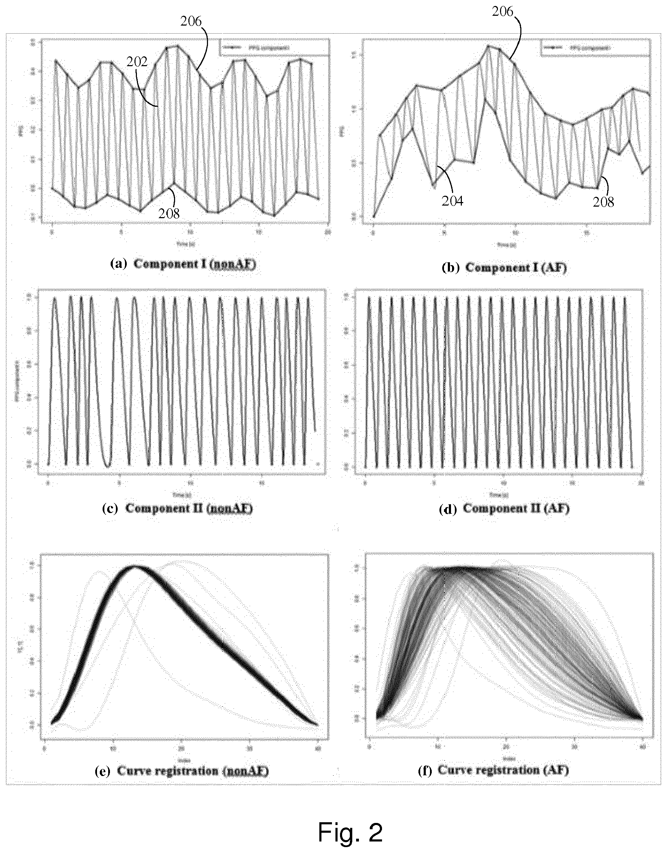

This is illustrated in Fig. 2a and 2b which show a PPG signal 202 with no atrial fibrillation (Fig. 2a) and a PPG signal 204 with atrial fibrillation (Fig. 2b). The long-term periodic component (e.g. "first component", component I or envelope) is modelled by fitting a spline function to the maxima (e.g. peaks) as shown by the line 206 and/or or minima (e.g. troughs) as shown by the line 208 of the individual pules in the PPG signals 202, 204.

In some embodiments, the step of decomposing and modelling 102 comprises modelling the short-term periodic component by performing curve registration, the curve registration comprising aligning curves corresponding to different pulses in the PPG signal.

In some embodiments the step of decomposing and modelling 102 may comprise decomposing the signal. For example, normalizing the amplitudes of pulses in the PPG signal. This is shown in Fig. 2c and 2d which show (amplitude) normalized versions of the pulses in Fig. 2a and 2b respectively. The PPG signal may be divided up into individual pulses at the maxima or minima of the pulses.

Curve registration may comprise superimposing or aligning the pulses. For example, curve registration may comprise performing a time transformation. As an example, curve registration may comprise aligning the maxima of the pulses. In some embodiments, curve registration may comprise aligning the maxima of the pulses in a common time window. The output of curve registration of the pulses is shown in Fig. 2e and 2f.

In some embodiments modelling the short-term periodic component comprises separately modelling each pulse in the PPG signal. For example, modelling or fitting a function separately to each individual pulse. In this way, the data points for each pulse may be summarized by the best fitting parameters and these may be stored instead of the full PPG data for each pulse, enabling each pulse in the PPG signal to be recreated without having to store the full dataset.

In some embodiments each pulse is modelled by fitting a non-parametric function to the pulse. In some embodiments each pulse is modelled by fitting a spline function (or a wavelet function) to each pulse. The skilled person will be familiar with other non-parametric functions that may be fit to the individual pulses. Fitting non-parametric functions such as splines may be more flexible and accurate compared to fitting parametric functions to each pulse of the PPG because parametric functions (e.g. such as a functions comprising one or more Gaussian profiles) assume a specific shape for the pulses which may not accurately reflect the shapes of real pulses of the PPG signal. The use of non-parametric functions is therefore more flexible and accurate because the real profile is modelled without any shape assumptions being made from the outset. This flexibility is important, for example, when modelling irregular PPG pulses, e.g. due to atrial fibrillation.

In some embodiments the pulses are modelled using a recursive procedure whereby information about previous pulses is used when fitting the next pulse. For example, the fit parameters (e.g. best fitting spline model) from a first pulse in a sequence of pulses may be used as input to the fitting procedure of the next pulse in the sequence.

In some embodiments the pulses are modelled using a Kalman filter.

In some embodiments an equation of a spline model in a state-space representation may be used to fit to each pulse, to enable the implementation of the Kalman filter.

In some embodiments modelling the short-term periodic component comprises modelling (e.g. recursively modelling) each pulse in the PPG signal using the following equations:

$$\begin{cases} \alpha_k = F\,\alpha_{k-1} + \varepsilon_k \\ Y_k = H_k\alpha_k + \eta_k \end{cases}$$

Here $\alpha_k$ is the vector of parameters that determine the shape of the kth pulse; $Y_k$ is the observed kth pulse and is modelled as a spline with shape parameters $\alpha_k$ plus some noise. Taking the shape parameter of the previous pulse into consideration in this way speeds up the fitting process and reduces the computational resources needed. Also, it takes into account the fact that on average the shape is not expected to significantly change. The spline model is flexible both in terms of fitting (e.g. to different shapes) and of choice of the number of parameters involved (e.g. more parameters and a better fit may be balanced against e.g. computational resources needed for the fitting process).

Turning now to the step 104 of Fig. 1, in some embodiments, summarizing 104 the information contained in the PPG signal may comprise summarizing (e.g. compressing) the PPG signal. This may comprise, for example, representing the PPG as a set of parameter values associated with the models of the long-term periodic component and/or the short-term periodic component, or the sets of (best fitting) parameter values for each pulse. By summarizing the PPG signal in this way, the PPG signal can be recreated (from the models and model parameters) without the need to store the (potentially high frequency and thus data rich) full data set of the PPG signal.

In some embodiments summarizing 104 the information contained in the PPG signal may comprise outputting a distribution of shape parameters, based on the modelled long-term and/or short-term periodic components of the PPG signal. The distribution of parameters may be presented, for example as a box-plot (as will be discussed below). The distribution may also be summarized using one or more statistical parameters, for example, statics such as the mean, median or standard deviation may be calculated.

In some embodiments summarizing 104 the information contained in the PPG signal may comprise analyzing one or more correlations between shape parameters of the modelled long-term and/or short-term periodic components of the PPG signal.

In some embodiments the method 100 may further comprise using the modelled long-term periodic component and/or the modelled short-term periodic component to monitor a patient and/or diagnose a disorder. For example, parameters obtained from the fitted splines (e.g. the best fit parameters) may be mapped (e.g. converted or calibrated) to one or more medical parameters.

The long-term periodic function (e.g. envelope) correlates with respiratory function and therefore, parameters obtained from modelling the long-term periodic function (e.g. the best fitting spline parameter values to the long-term periodic component or envelope) may be mapped (e.g. converted or calibrated) onto one or more medical parameters. For example, medical parameters related to respiration such as respiration rate, flow rate or $CO_2$.

The short-term periodic component can be used to determine medical parameters relating to cardiac function. For example, the parameters obtained from modelling the short-term periodic function (e.g. the best fitting spline parameter values to the individual pulses) may be mapped (e.g. converted or calibrated) onto one or more medical parameters such as, for example pulse amplitude and inter-beat intervals (IBIs), amongst others. In this way, the model of the long-term periodic component and the model of the short-term periodic component allows the information extracted from the PPG signal to be linked to physiological information.

In some embodiments, cardiac parameters may be further used to diagnose a disorder or medical condition (e.g. via the determined physiological information above). For example, such as detecting irregular heart rhythms (e.g. arrhythmia such as atrial fibrillation).

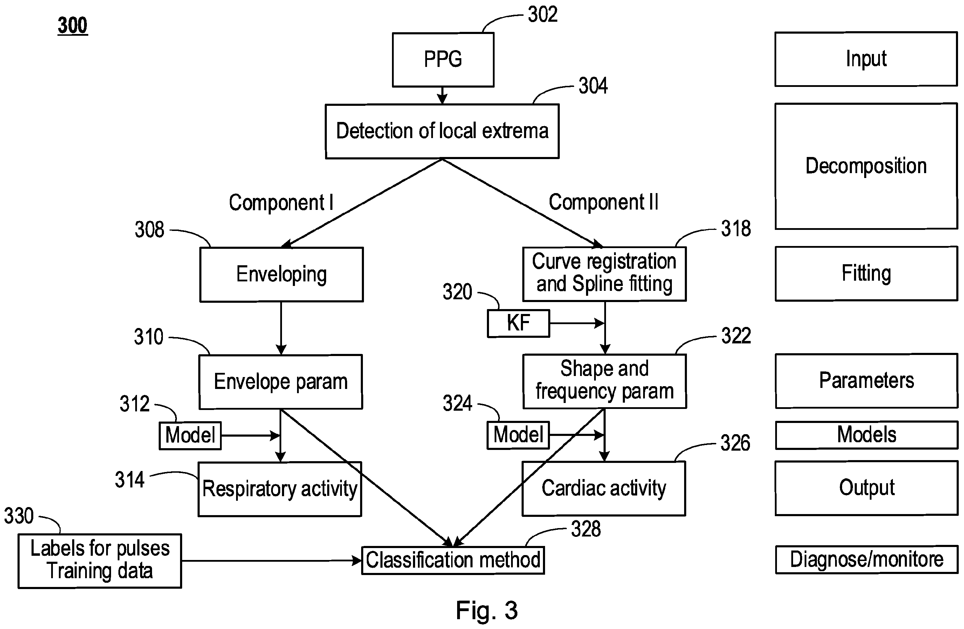

Turning now to Fig. 3, Fig. 3 shows a method 300 according to an embodiment herein. In a first step 302, a PPG signal is acquired. The PPG signal may be acquired by taking measurements from a patient for example, or acquired from a database of PPG signals.

In a step 304 the method may comprise determining the location of local minima and maxima of the PPG signal (e.g. local extrema of the PPG signal). The skilled person will be familiar with methods for determining minima and maxima of a signal.

In steps 308-310 and 318-326, the method 300 comprises decomposing and modelling the PPG signal as a long-term periodic component and a short-term periodic component. Decomposing and modelling the PPG signal as a long-term periodic component and a short-term periodic component was described above with respect to step 102 of Fig. 1 and the details therein will be understood to apply to this embodiment.

In more detail, in steps 308-310, the long-term periodic component (e.g. component I or first component) is modelled by determining in step 308 an envelope of the PPG signal. The envelope may be modelled by, for example, fitting a spline function to the local maxima of the PPG signal as determined in step 304 (or the local minima). In step 310, fitted parameters of the modelled first component (e.g. envelope) are determined.

In steps 318-322, the short-term periodic component (e.g. component II or second component) is modelled. In step 318, curve registration is performed and individual splines are fitted to each pulse in the PPG signal, e.g. using a Kalman filter 320 as described above. Curve registration and fitting splines to individual pulses was described above with respect to step 102 of the method 100 and the details therein will be understood to apply to step 318 here. Shape and frequency parameters, derived from the best filling models may be determined in step 322. In some embodiments frequency parameters may be obtained from the local extrema and timing information.

Once the PPG signal is decomposed and modelled as a long-term periodic component and a short-term periodic component, the distribution of fitted parameters 310 of the modelled long-term periodic components may be used along with the respective model 312 (e.g. the model that was fit to the long-term periodic component) to determine respiratory activity in a step 314. For example, the fitted parameters 310 may be calibrated to clinical parameters such as, for example, respiration rate.

The distribution of fitted parameters 322 (e.g. shape and frequency parameters) of the modelled short-term periodic components may be used along with the respective model 324 (e.g. the model that was used to fit to the short-term periodic components) to determine cardiac activity in a step 326. For example, the fitted parameters 320 may be calibrated to clinical parameters such as, for example, heart rate or a measure of arrhythmia or atrial fibrillation.

The envelope parameters 310 for the long-term periodic component and the shape and frequency parameters 322 for the short-term periodic components may be complemented by labels for the pulses to classify the nature of each pulse based on the distribution of the fitted parameters 310, 322. When a new (set of) PPG pulse(s) is acquired, the respective parameters (e.g. modelled parameters) can be compared with the shape parameters of past pulses to detect statistically significant deviations. Summarizing the information contained in the PPG signal was described with respect to step 104 of method 100 above and the details therein will also be understood to apply here.

Turning now to other embodiments, in some embodiments there is a system configured to carry out any of the embodiments of the methods 100 or 300 described above.

In some embodiments there is a system comprising a processor, the processor being configured to perform any of the methods 100 or 300 described above.

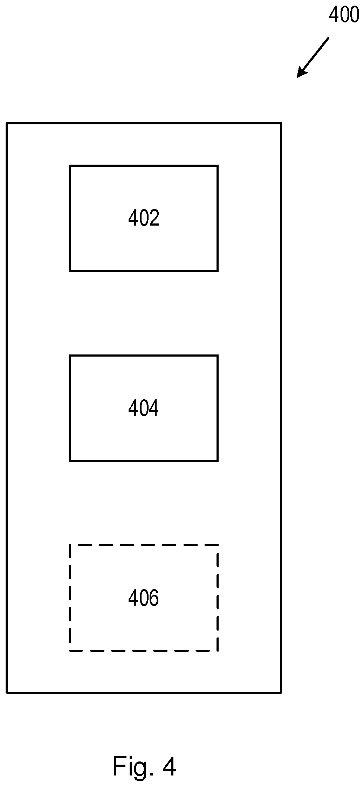

In more detail and turning now to Fig. 4, according to some embodiments there is a system 400 configured for modelling and extracting information from a photoplethysmography, PPG, signal.

In some embodiments, the system 400 may comprise a memory 404 and a processor 402. The memory may comprise instruction data representing a set of instructions. The processor 402 may be configured to communicate with the memory 404 and to execute the set of instructions. The set of instructions when executed by the processor may cause the processor to perform any of the embodiments of the methods 100 or 300 as described above. The memory 404 may be configured to store the instruction data in the form of program code that can be executed by the processor 402 to perform the method 100 described above.

In some implementations, the instruction data can comprise a plurality of software and/or hardware modules that are each configured to perform, or are for performing, individual or multiple steps of the method described herein. In some embodiments, the memory 404 may be part of a device that also comprises one or more other components of the system 400 (for example, the processor 402 and/or one or more other components of the system 400). In alternative embodiments, the memory 404 may be part of a separate device to the other components of the system 400.

In some embodiments, the memory 404 may comprise a plurality of sub-memories, each sub-memory being capable of storing a piece of instruction data. In some embodiments where the memory 404 comprises a plurality of sub-memories, instruction data representing the set of instructions may be stored at a single sub-memory. In other embodiments where the memory 404 comprises a plurality of sub-memories, instruction data representing the set of instructions may be stored at multiple sub-memories. Thus, according to some embodiments, the instruction data representing different instructions may be stored at one or more different locations in the system 400. In some embodiments, the memory 404 may be used to store information, such as the PPG signal, the models used in the step of decomposing and modelling, parameters associated with the models or any other information output or used to perform the method 100.

The processor 402 can comprise one or more processors, processing units, multi-core processors and/or modules that are configured or programmed to control the system 400 in the manner described herein. In some implementations, for example, the processor 402 may comprise a plurality of (for example, interoperated) processors, processing units, multi-core processors and/or modules configured for distributed processing. It will be appreciated by a person skilled in the art that such processors, processing units, multi-core processors and/or modules may be located in different locations and may perform different steps and/or different parts of a single step of the method described herein.

Briefly, the set of instructions, when executed by the processor 402, cause the processor 402 to decompose and model the PPG signal as a long-term periodic component and a short-term periodic component, and summarize the information contained in the PPG signal, based on a distribution of fitted parameters of the modelled long-term and short-term periodic components.

In some embodiments the system 400 may further comprise a PPG sensor 406 (e.g. such as a pulse oximeter) and the processor 402 may further be configured to acquire the PPG measurements using the PPG sensor 406.

It will be appreciated that the system 400 may comprise additional components to those illustrated in Fig. 4. The system 400 may further comprise one or more user interfaces such as a display screen, mouse, keyboard or any other user interface allowing information to be displayed to a user or input to be received from a user. In some embodiments, the system 400 may further comprise a power source such as a battery or mains power connection.

According to further embodiments, there is a computer program product comprising a non-transitory computer readable medium, the computer readable medium having computer readable code embodied therein, the computer readable code being configured such that, on execution by a suitable computer or processor, the computer or processor is caused to perform the method 100.

The description below illustrates some further examples according to some embodiments of the methods and apparatus described herein.

The main element(s) of an example embodiment

According to an embodiment there is a two-step approach decomposing the signal into the long- and short-term periodicity components, and then modelling the two. The first component is modelled by interpolating certain features of the signal using (two) piecewise (linear) splines and is shown to be highly correlated with respiratory activity. The remaining detrended signal is modelled through a recursive procedure, allowing the inclusion of the relevant past information. Each pulse is modelled separately (e.g. sequentially), first performing curve registration and then fitting a spline function. Decomposing and modelling the PPG signal was described above with respect to step 102 of method 100 and Fig. 1 and the details therein will be understood to apply equally to this embodiment.

As a result, the PPG is then summarized by the shape parameters and variances of each modelled component. The PPG may further be summarized by the time transformation of each model component. Summarizing the PPG signal was described above with respect to step 104 of method 100 and Fig. 1 and the details therein will be understood to apply equally to this embodiment.

The embodiment provides structures to preprocess, model and interpret PPG, possibly in combination with other devices (e.g. ECG, respirometer). This knowledge can be contrasted to data from patients with various disorders (e.g. arrhythmias, heart failure, arterial stiffness) and medications to explain the differences observed in PPGs, possibly in combination with other measurements.

Examples of various features of some embodiments:

     1. The system is self-contained
     2. The method can be implemented in real time
     3. The system is fully automated
     4. A method for deep understanding of PPG signals, possibly in combination with information from other devices (e.g. accelerometer, respirometer, ECG)
     5. A model for PPG signals fully based on splines
     6. A model-based estimate of respiratory rate
     7. A system performing curve registration
     8. The system can be tuned to trade-off compression and accuracy
     9. A system to diagnose various conditions (e.g. arrhythmias, heart failure, arterial stiffness)
     10. A system to monitor (e.g. healthy people, patients with different conditions)
     11. A model-based approach providing the distribution of the shape of PPG pulses and their variation over time (e.g. distribution and correlations between shape parameters)
     12. A system performing a time transformation and storing this information for analysis/diagnosis.

NB. In the following, we make use of the abbreviations AF for atrial fibrillation and nonAF for non atrial fibrillation.

Detailed description of how to build and use the example embodiment

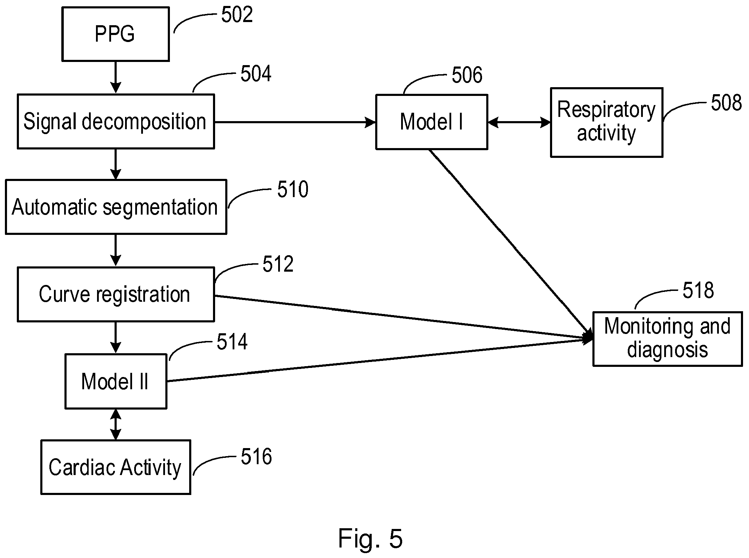

In Fig 5 we show the flowchart of the invention according to the example embodiment. The PPG signal 502 is initially automatically decomposed into its two main components 504. Component I (e.g. Fig 2a for nonAF, Fig 2b for AF) captures, among other information, the baseline wandering, the change in amplitude and the long-term periodicity. This envelope is then modelled via spline functions (Model I 506). The results are shown to correlate significantly with respiratory information 508 (i.e. flow, $CO_2$). (Note: Decomposing and modelling using spline functions was described above with respect to step 104 of the method 100 and the details therein will be understood to apply equally to the embodiment described here.)

The processed PPG constitutes then component II (Fig 2c for nonAF, Fig 2d for AF). The system performs here automatic segmentation 510 into the single pulses and then curve registration 512 to align all the pulses to the interval [0, 1] (Fig 2e for nonAF, Fig 2f for AF). (Note: Curve registration was described above with respect to method 100 of Fig. 1 and the details therein will be understood to apply the embodiment described here.) The composition function of the registration step provides information on the variability of pulse lengths. The registered curves are then modelled by mean of spline functions while keeping into account the implicit periodicity (Model II. 514 Efficient estimation via Kalman filter, considering only the relevant past information). The outcome of this model gives information, among the rest, about the shape of the PPG pulses and their variability over time. Also information related to cardiac activity 516. The outcomes of the models can be monitored over to time for monitoring and diagnosis purposes 518.

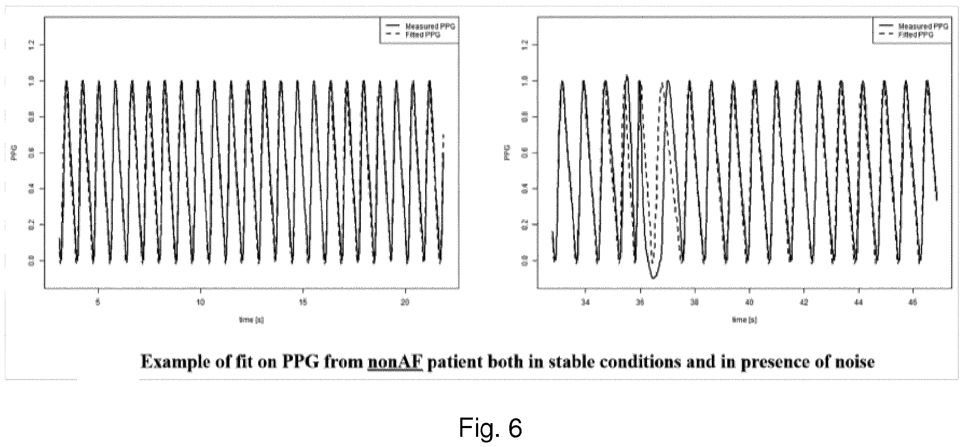

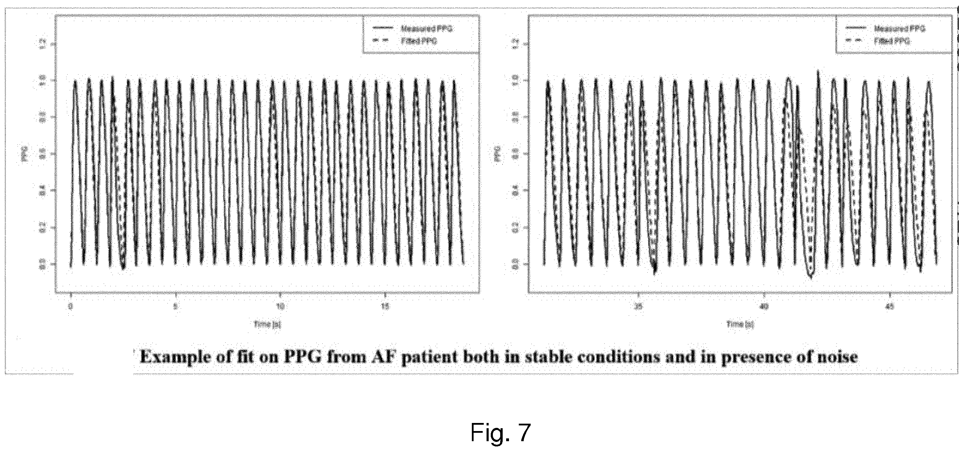

The algorithm produces an improved fit in patients without atrial fibrillation and is robust to noisy segments (see an example of fit to Component II from the PPG of a patient without atrial fibrillation, but with other morbidities Fig 6). At the same time, it provides an improved compression of the data. The user can choose for the trade-off between accuracy and compression by tuning the modelling parameters to achieve the desired goal. Also in patients with atrial fibrillation the model fit is better, readapting quickly after rhythm irregularities (see an example of fit to Component II from the PPG of a patient with atrial fibrillation, Fig 7).

Uses

The example embodiment describes the information contained in the PPG both via the models for Component I and Component II, and can be used to monitor people/patients and diagnose disorders.

An example of application of the model is to distinguish between patients with and without atrial fibrillation (AF, nonAF). Information derived from the signal of a patient supposed to have atrial fibrillation can be contrasted to the information extracted from a person without this condition.

Further example embodiment

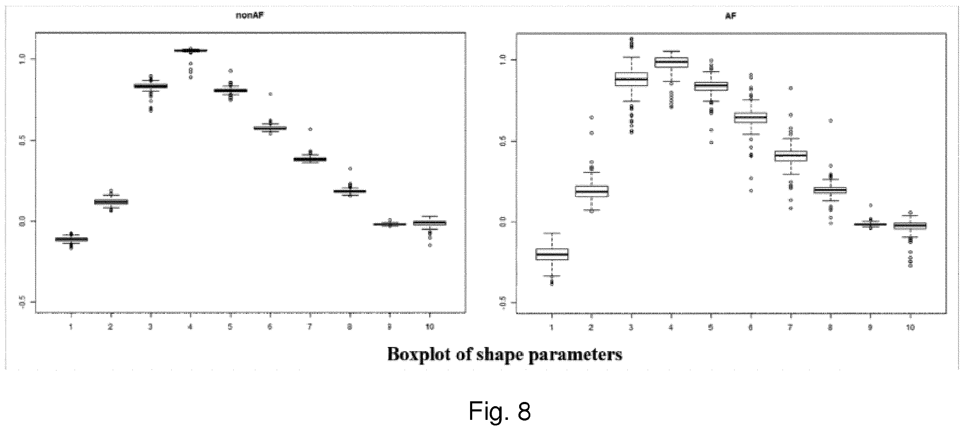

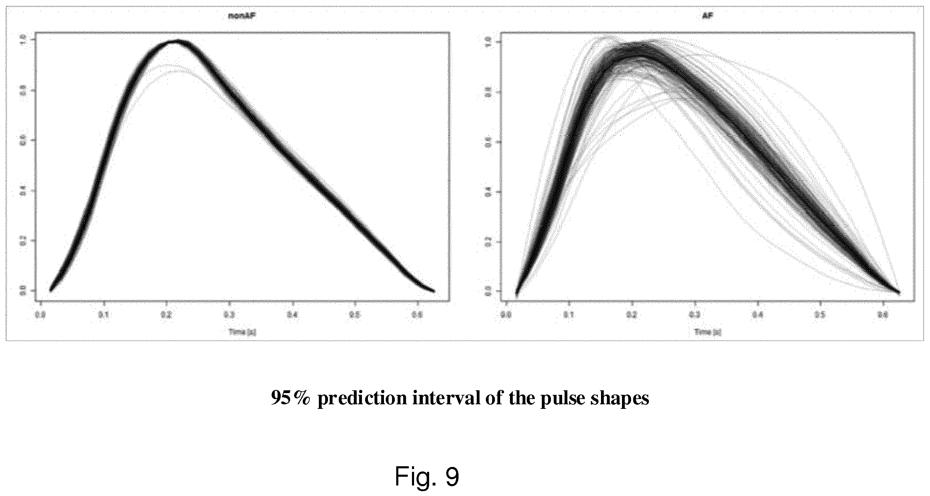

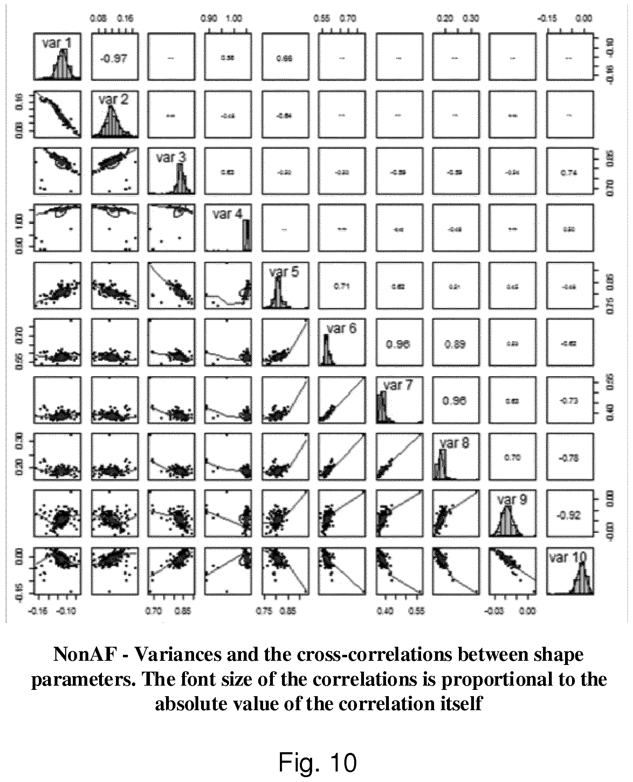

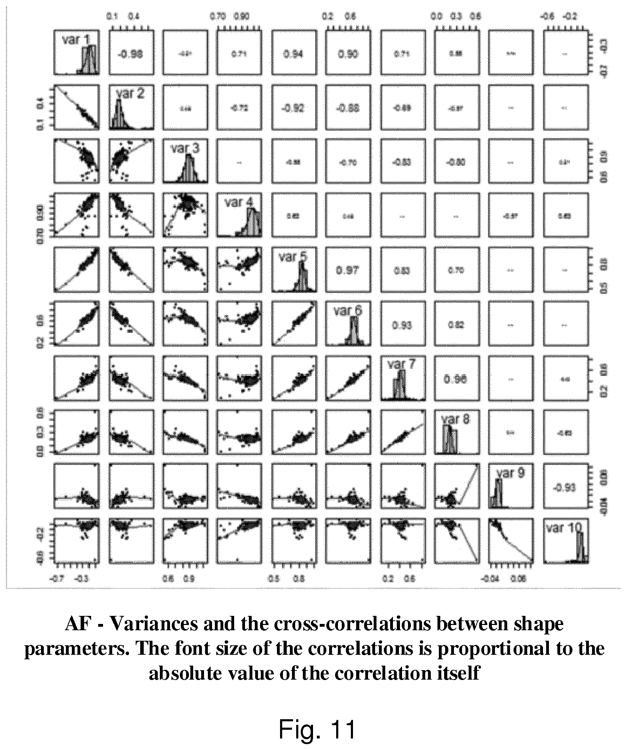

The boxplot of the shape parameters (Fig 8, obtained from fitting 200 pulses from a nonAF (left) and an AF patient (right) respectively) is an example of possible summary of the information provided by Model II (e.g. an output of the step of summarizing 104 described above with respect to Fig. 1). Information on the shape is provided also by the 95% prediction interval of the predicted pulses shape (Fig 9, obtained again from fitting 200 pulses from a nonAF (left) and an AF patient (right) respectively). Also the variances and the crosscorrelations (e.g. between shape parameters) (Fig 10 for nonAF and Fig 11 for AF, obtained again from fitting 200 pulses from a nonAF (left) and an AF patient (right) respectively) between shape parameters provide nontrivial information, with insight in the variability of the shapes across time and the corresponding relationship between shape parameters in patients with various disorders or conditions.

Applications

Application is intended for various kinds of potential patients and possibly implemented to a wrist watch for continuous or all day monitoring.

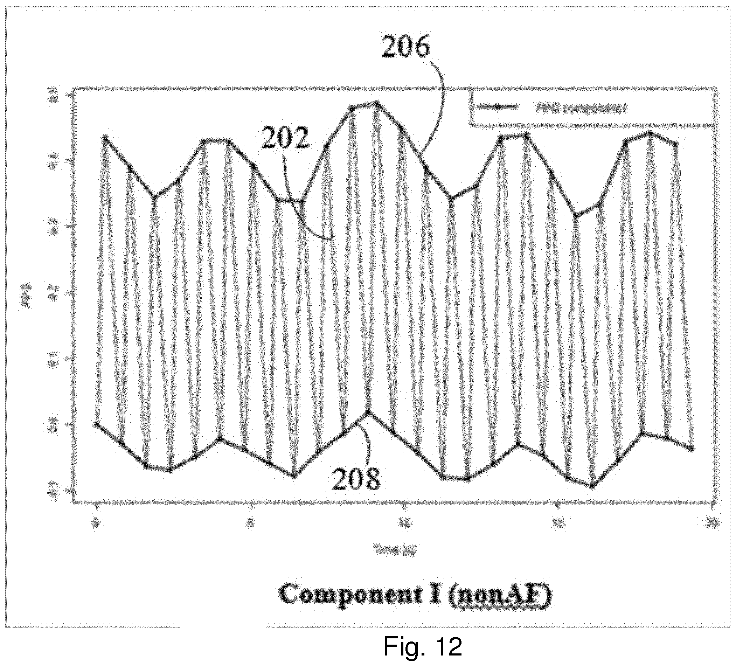

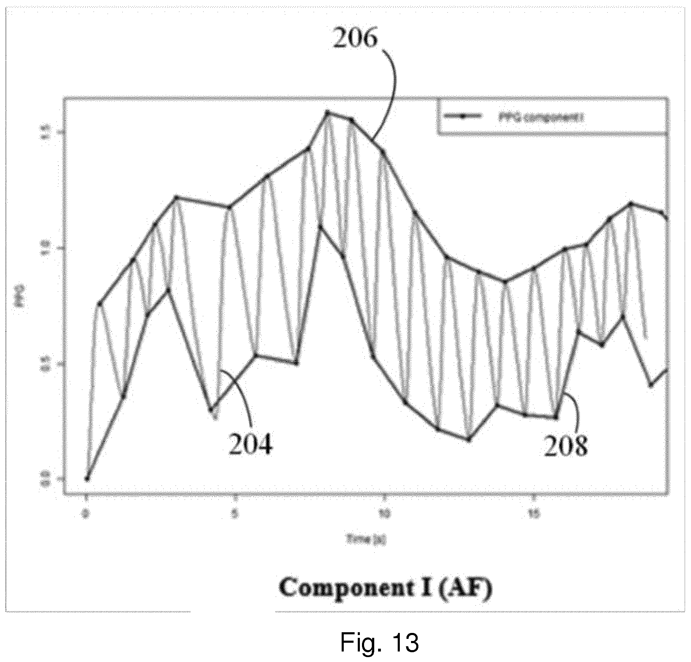

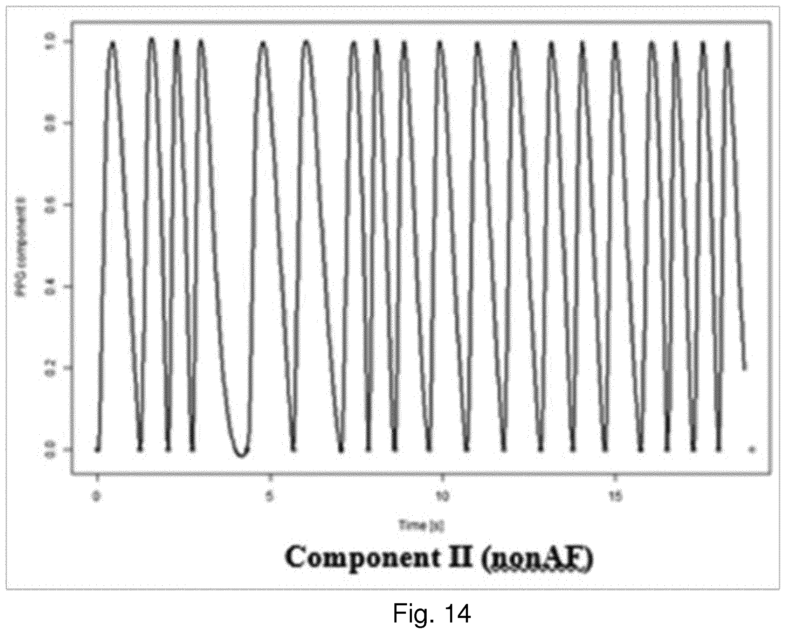

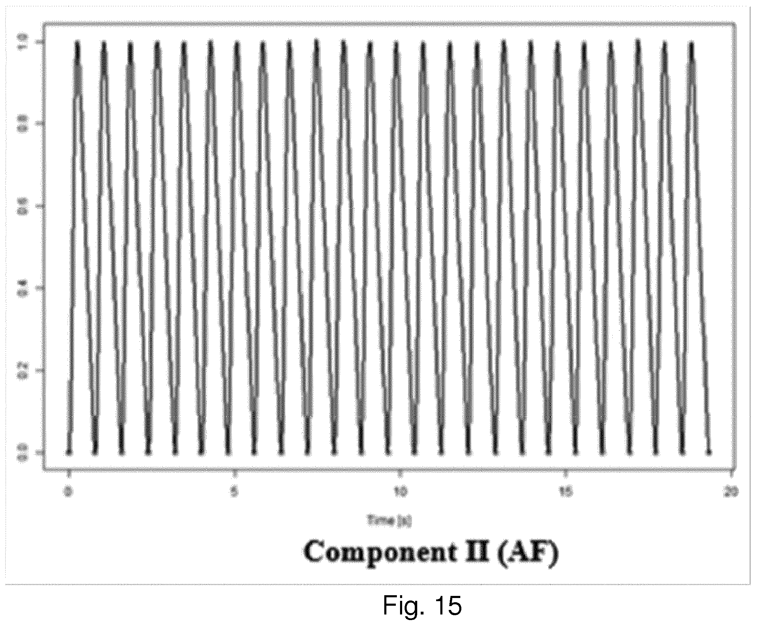

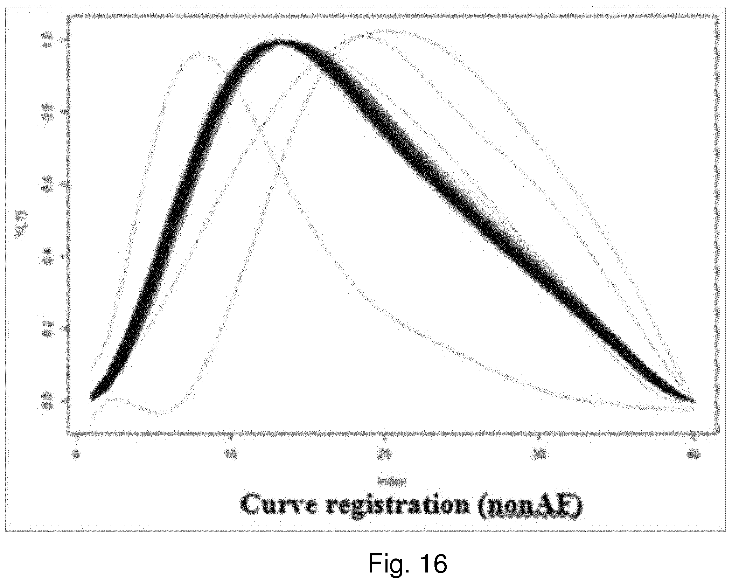

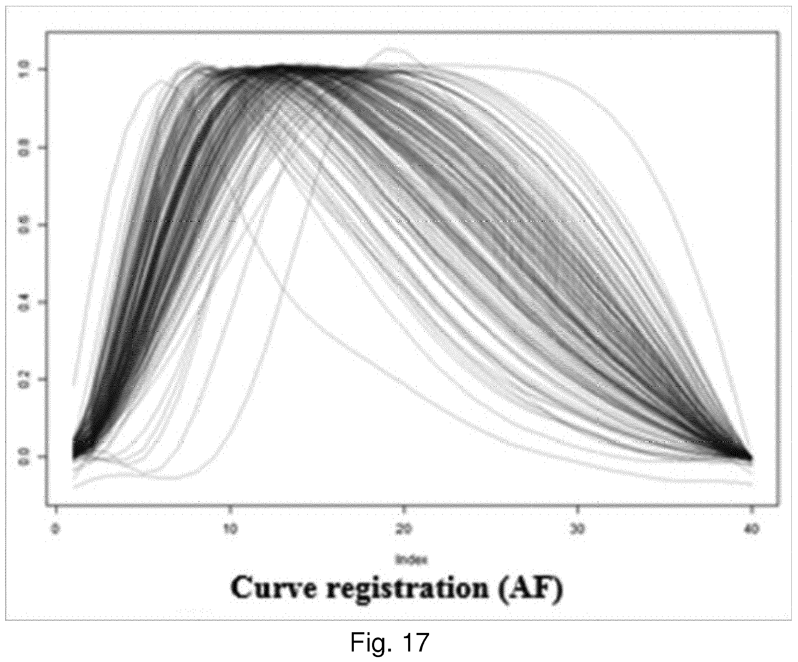

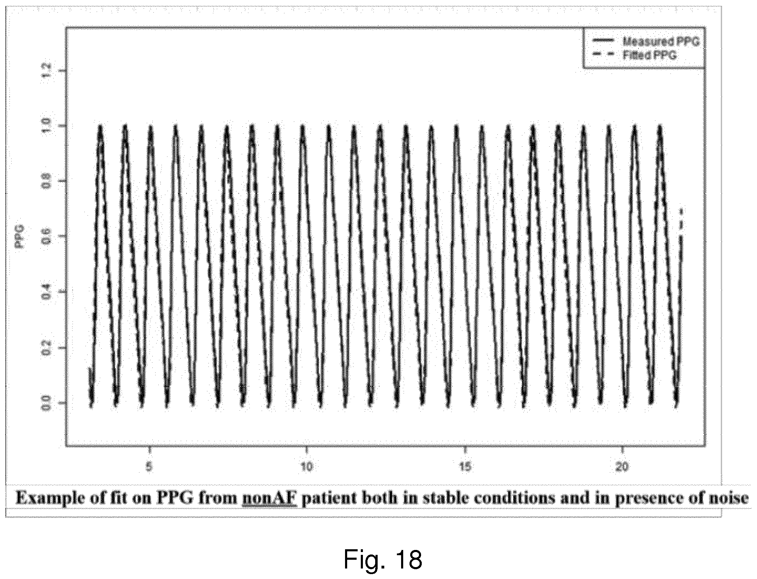

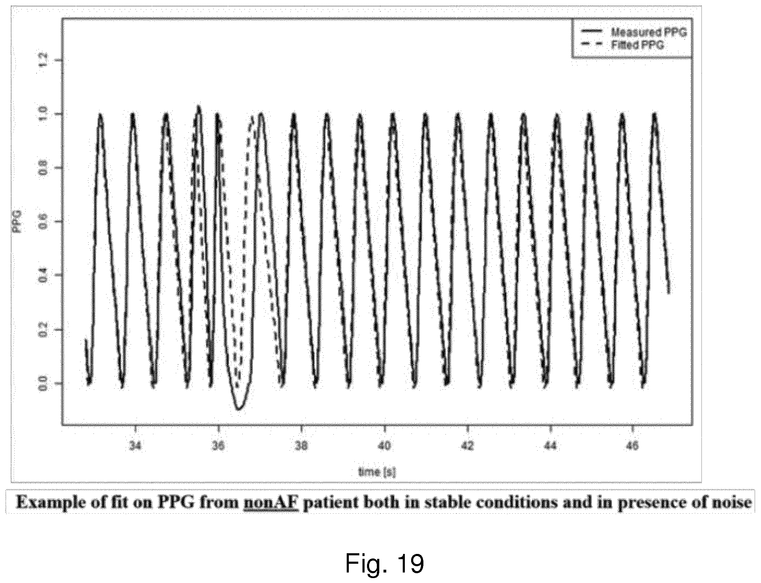

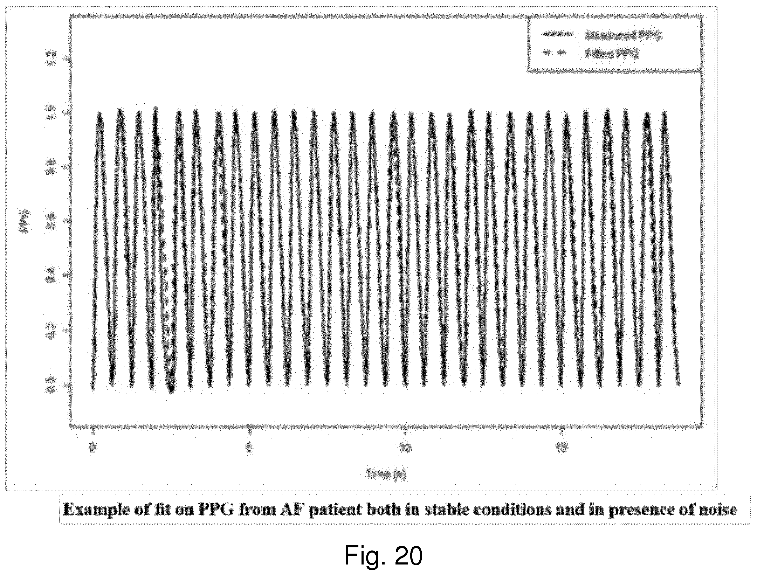

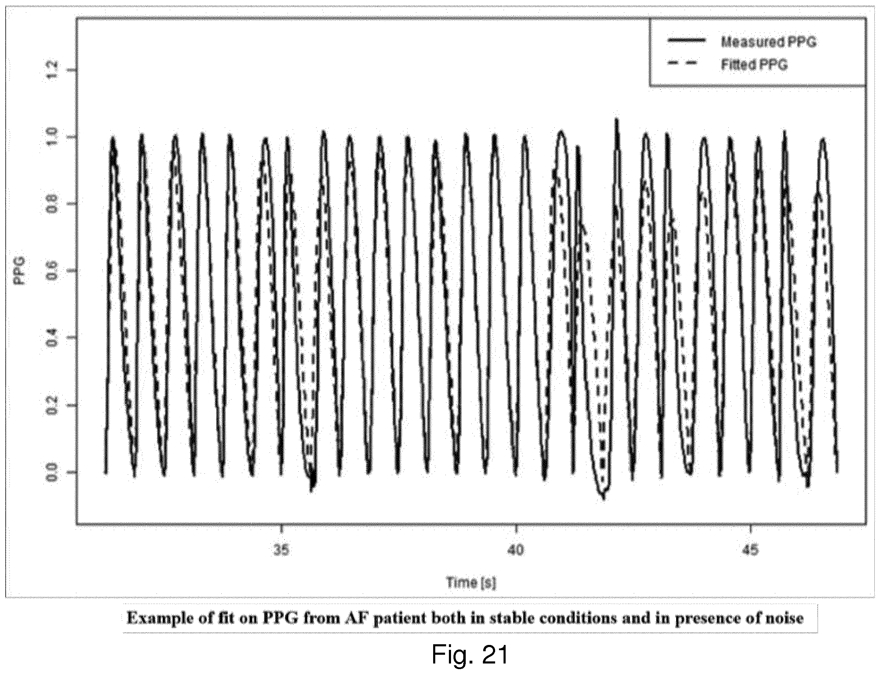

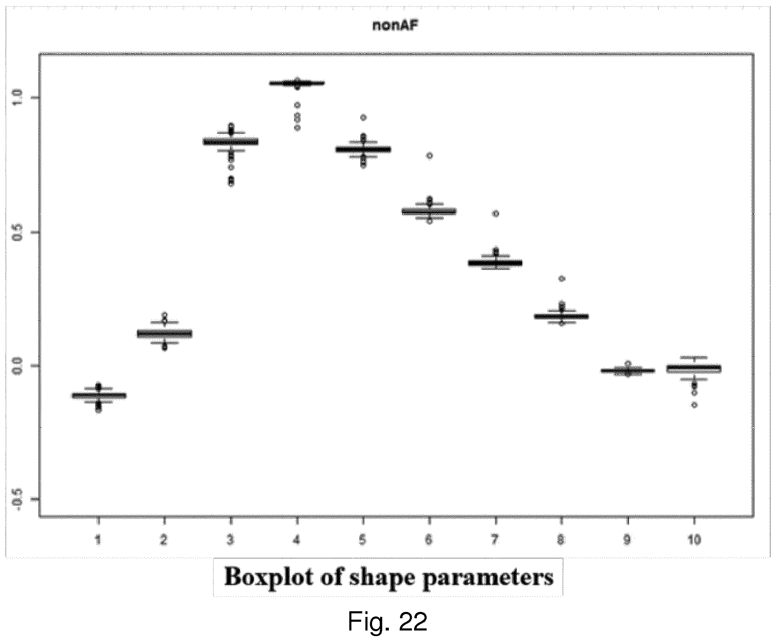

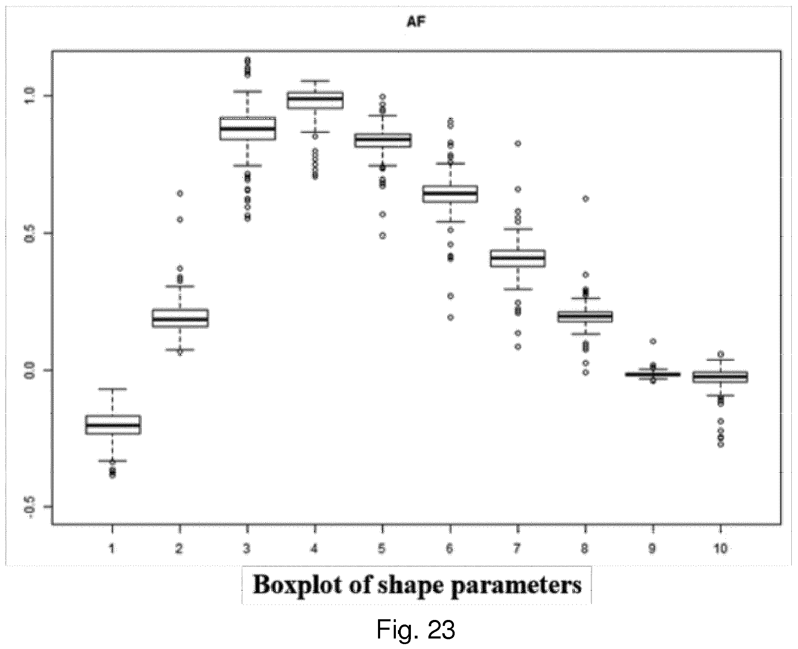

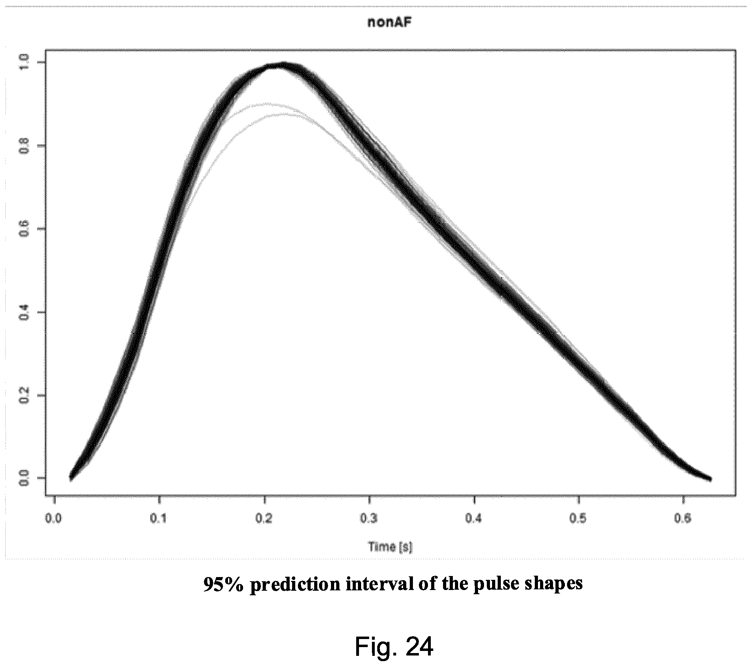

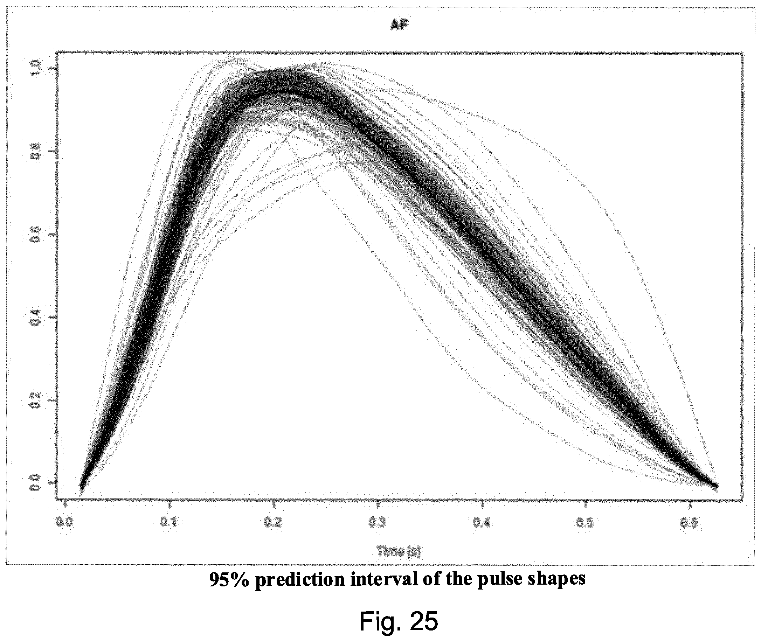

For completeness, Figs 2a-f, 6, 7, 8 and 9 are reproduced in larger form in Figs 11-25 respectively.

The methods and apparatus described herein may provide improved models for PPG signals (including PPG signals with or without atrial fibrillation) and furthermore returns a summary of the data that can be used for diagnostic purposes.

It will be appreciated that the embodiments of the invention also apply to computer programs, particularly computer programs on or in a carrier, adapted to put the invention into practice. The program may be in the form of a source code, an object code, a code intermediate source and an object code such as in a partially compiled form, or in any other form suitable for use in the implementation of the method according to embodiments of the invention. It will also be appreciated that such a program may have many different architectural designs. For example, a program code implementing the functionality of the method or system according to the invention may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other. An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

Bibliography

Dekker, A. (2003). Patent No. WO2003071938 A1. Retrieved 11 02, 2017, from https://www.google.nl/patents/WO2003071938A1?cl=en

Pimentel, M., Charlton, P., & Clifton, D. (2015). Probabilistic estimation of respiratory rate from wearable sensors. Wearable electronics sensors, 241-262.

Tarassenko, L., & Fleming, S. (2009). Patent No. WO2009016334 A1. Retrieved 11 02, 2017, from https://www.google.nl/patents/WO2009016334A1?cl=en&dq=modeling+ppg+signal&hl=nl&sa=X&ved=0ahUKEwjH3uGsZDXAhXKY1AKHWYhAtkQ6AEILzAB Townsend, N., & Collins, S. (2003). Patent No. WO2003005893 A2. Retrieved from https://www.google.nl/patents/WO2003005893A2?cl=en

**Claims**

1. A computer implemented method of modelling and extracting information from a photoplethysmography, PPG, signal, the method comprising:

   decomposing and modelling 102 the PPG signal as a long-term periodic component and a short-term periodic component; and
   summarizing 104 the information contained in the PPG signal, based on a distribution of fitted parameters of the modelled long-term and short-term periodic components.

2. A method as in claim 1 wherein the step of decomposing and modelling 102 comprises:

   modelling the short-term periodic component by performing curve registration, the curve registration comprising aligning curves corresponding to different pulses in the PPG signal.

3. A method as in claim 1 or 2 wherein modelling the short-term periodic component comprises:

   separately modelling each pulse in the PPG signal.

4. A method as in claim 3 wherein each pulse is modelled by fitting a non-parametric function to the pulse.

5. A method as in claim 3 or 4 wherein each pulse is modelled by fitting a spline function to the pulse.

6. A method as in claim 3, 4 or 5 wherein the pulses are modelled using a recursive procedure whereby information about a previous pulse is used when fitting the next pulse.

7. A method as in any one of claims 3 to 6 wherein the pulses are modelled using a Kalman filter.

8. A method as in any one of claims 1 to 7 wherein the long-term periodic component comprises an envelope of the PPG signal.

9. A method as in any one of claims 1 to 8 wherein the step of decomposing and modelling 102 comprises:

   modelling the long-term periodic component using spline functions.

10. A method as in claim 9 wherein the step of decomposing and modelling 102 comprises:

    fitting the spline functions to one or more maxima or one or more minima of individual pules in the PPG signal.

11. A method as in any one of claims 1 to 10 further comprising:

    outputting a distribution of shape parameters, based on the modelled long-term and/or short-term periodic components of the PPG signal.

12. A method as in any one of claims 1 to 11 further comprising:

    analysing one or more correlations between shape parameters of the modelled long-term and/or short-term periodic components of the PPG signal.

13. A method as in any one of claims 1 to 12 further comprising:

    using the modelled long-term periodic component and/or the modelled short-term periodic component to monitor a patient and/or diagnose a disorder.

14. A system configured for modelling and extracting information from a photoplethysmography, PPG, signal, the system being configured to:

    decompose and model the PPG signal as a long-term periodic component and a short-term periodic component; and
    summarize the information contained in the PPG signal, based on a distribution of fitted parameters of the modelled long-term and short-term periodic components.

15. A computer program product comprising a non-transitory computer readable medium, the computer readable medium having computer readable code embodied therein, the computer readable code being configured such that, on execution by a suitable computer or processor, the computer or processor is caused to perform the method of any one of claims 1 to 13.

100

102

104

Fig. 1

(a) Component I (nonAF)

(b) Component I (AF)

(c) Component II (nonAF)

(d) Component II (AF)

(e) Curve registration (nonAF)

(f) Curve registration (AF)

Fig. 2

**300**

Fig. 3

400

402

404

406

Fig. 4

Fig. 5

EP 3 527 122 A1

**Example of fit on PPG from _nonAF_ patient both in stable conditions and in presence of noise**

Fig. 6

Fig. 7

Example of fit on PPG from AF patient both in stable conditions and in presence of noise

Boxplot of shape parameters

Fig. 8

**95 % prediction interval of the pulse shapes**

Fig. 9

**NonAF - Variances and the cross-correlations between shape parameters. The font size of the correlations is proportional to the absolute value of the correlation itself**

Fig. 10

**AF - Variances and the cross-correlations between shape parameters. The font size of the correlations is proportional to the absolute value of the correlation itself**

Fig. 11

Component I (nonAF)

Fig. 12

Component I (AF)

Fig. 13

EP 3 527 122 A1

Component II (nonAF)

Fig. 14

Component II (AF)

Fig. 15

Curve registration (nonAF)

Fig. 16

Curve registration (AF)

Fig. 17

Example of fit on PPG from nonAF patient both in stable conditions and in presence of noise

Fig. 18

**Example of fit on PPG from nonAF patient both in stable conditions and in presence of noise**

Fig. 19

**Example of fit on PPG from AF patient both in stable conditions and in presence of noise**

Fig. 20

EP 3 527 122 A1

Example of fit on PPG from AF patient both in stable conditions and in presence of noise

Fig. 21

Boxplot of shape parameters

Fig. 22

EP 3 527 122 A1

**Boxplot of shape parameters**

Fig. 23

nonAF

Time [s]

**95% prediction interval of the pulse shapes**

Fig. 24

95% prediction interval of the pulse shapes

Fig. 25

Europäisches
Patentamt

European
Patent Office

Office européen
des brevets

# EUROPEAN SEARCH REPORT

Application Number

EP 18 15 6677

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JAAFAR ROSMINA ET AL: "Estimation of breathing rate and heart rate from photoplethysmogram", 2017 6TH INTERNATIONAL CONFERENCE ON ELECTRICAL ENGINEERING AND INFORMATICS (ICEEI), IEEE, 25 November 2017 (2017-11-25), pages 1-4, XP033329448, DOI: 10.1109/ICEEI.2017.8312414 [retrieved on 2018-03-09] * the whole document * | 1-15 | INV. A61B5/00 A61B5/024 |
| A | XUXUE SUN ET AL: "Robust heart beat detection from photoplethysmography interlaced with motion artifacts based on Empirical Mode Decomposition", BIOMEDICAL AND HEALTH INFORMATICS (BHI), 2012 IEEE-EMBS INTERNATIONAL CONFERENCE ON, IEEE, 5 January 2012 (2012-01-05), pages 775-778, XP032449184, DOI: 10.1109/BHI.2012.6211698 ISBN: 978-1-4577-2176-2 * the whole document * | 1-15 | |
| A | US 2010/014724 A1 (WATSON JAMES NICHOLAS [GB] ET AL) 21 January 2010 (2010-01-21) * paragraph [0038] * * paragraph [0045] * * paragraph [0057] - paragraph [0095] * * figures 1-7 * | 1-15 | TECHNICAL FIELDS SEARCHED (IPC) A61B |

The present search report has been drawn up for all claims

| Place of search | Date of completion of the search | Examiner |
|---|---|---|
| The Hague | 30 July 2018 | Marteau, Frédéric |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 15 6677

This annex lists the patent family members relating to the patent documents cited in  the above-mentioned European search report.
The members are as contained in the European Patent Office EDP file on
The European Patent Office is in no way liable for these particulars which are merely given for the purpose of information.

30-07-2018

| Patent document cited in search report | | Publication date | Patent family member(s) | | Publication date |
|---|---|---|---|---|---|
| US 2010014724 | A1 | 21-01-2010 | AU | 2009272391 A1 | 21-01-2010 |
| | | | CA | 2730613 A1 | 21-01-2010 |
| | | | EP | 2339956 A1 | 06-07-2011 |
| | | | US | 2010014724 A1 | 21-01-2010 |
| | | | US | 2012278001 A1 | 01-11-2012 |
| | | | US | 2012283536 A1 | 08-11-2012 |
| | | | WO | 2010007486 A1 | 21-01-2010 |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2003071938 A1, Dekker, A.
- WO 2009016334 A1, Tarassenko, L., & Fleming, S.
- WO 2003005893 A2, Townsend, N., & Collins, S.

**Non-patent literature cited in the description**

- **PIMENTEL, M. ; CHARLTON, P. ; CLIFTON, D.** Probabilistic estimation of respiratory rate from wearable sensors. *Wearable electronics sensors,* 2015, 241-262